(19) Europäisches Patentamt
European Patent Office
Office européen des brevets

(11) Publication number: 0 272 007 B1

(12) # EUROPEAN PATENT SPECIFICATION

(45) Date of publication of patent specification: **04.03.92**

(51) Int. Cl.[5]: **C09B 11/24**

(21) Application number: **87310256.0**

(22) Date of filing: **20.11.87**

The file contains technical information submitted after the application was filed and not included in this specification

(54) **5- and 6- Succinimidyl - carboxylate isomers of rhodamine dyes.**

(30) Priority: **15.12.86 US 941985**

(43) Date of publication of application:
**22.06.88 Bulletin 88/25**

(45) Publication of the grant of the patent:
**04.03.92 Bulletin 92/10**

(84) Designated Contracting States:
**DE FR GB SE**

(56) References cited:
**FR-A- 2 089 774**
**GB-A- 2 051 849**
**GB-A- 2 059 984**
**US-A- 4 745 181**

**FIESER & FIESER Vol. 9, p 208 (1981)**

**D J Brunelle. Tetrahedron Letters, Vol. 23, No. 1739 (1982)**

**H Ogura Tetrahedron Letters No.49, 4745 (1979)**

(73) Proprietor: **APPLIED BIOSYSTEMS, INC.**
**850 Lincoln Centre Drive**
**Foster City California 94404(US)**

(72) Inventor: **Menchen, Steven M.**
**768 Vanda Way Fremont**
**CA 94536(US)**
Inventor: **Fung, Steven**
**765 San Antonio Road No. 8, Palo Alto**
**CA 94303(US)**

(74) Representative: **West, Alan Harry et al**
**R.G.C. Jenkins & Co. 26 Caxton Street**
**London SW1H 0RJ(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates generally to fluorescent probes and/or compounds for labeling molecules of biological or biochemical interest, and more particularly, to N-hydroxysuccinimide activated 5- and 6-carboxyl isomers of rhodamine dyes.

Fluorescent labels find widespread applications in molecular biology, microbiology, biochemistry, analytical chemistry, and in the industrial and medical fields rooted in those disciplines, e.g. biotechnology, and environmental, industrial, and diagnostic medicine.

A large number of organic and inorganic fluorescent materials are available. However, many problems are encountered in attempts to construct fluorescent labels out of such materials. The term "fluorescent label" as used herein means an organic molecule having a fluorescent moiety and a linking moiety, such that the linking moiety allows the fluorescent moiety to be covalently attached to a target functionality, e.g. primary amines, secondary amines, or the like.

Some important considerations associated with the construction and use of fluorescent labels include the stability of the bond between the fluorescent moiety and the linking moiety, the effect that the linking moiety has on fluorescent characteristics before and after attachment to a target functionality, the stability of the bond between the fluorescent moiety and the target functionality, i.e. the strength of the linking moiety after attachment, the stability of the linking functionality to purification procedures, e.g. amide linkages are more stable to hydrolysis than thiourea linkages, the reactivity of the linking functionality with the target functionality, and the like. When multiple fluorescent labels are required, each having distinct fluorescent characteristics, such as nonoverlapping emission bands, selection of labels for a particular application can become very difficult, often necessitating tradeoffs between the desired characteristics of the available fluorescent labels.

When fluorescent labels are used to track target molecules in biochemical separation procedures or in biological processes, two important factors are (1) the extent to which the fluorescent label perturbs the behavior of the target molecules, and (2), if such a perturbation does exist, the uniformity of the perturbation as between target molecules. This latter factor is especially crucial in methods for separating macromolecules on the basis of size by gel electrophoresis. In particular, techniques for sequencing deoxyribonucleic acids (DNAs) rely heavily on the ability to separate oligonucleotides differing by no more than a single base, e.g. Smith et al., Nucleic Acids Research, Vol. 13, pgs. 2399-2412 (1985); Schreier et al., J. Mol. Biol., Vol. 129, pgs. 169-172 (1979); and Sanger et al., J. Mol. Biol., Vol. 143, pgs. 161-178 (1980).

Recently, improvements in such methodologies have made use of multiple fluorescent labels to carry out sequencing automatically on a single columnar gel, e.g. Smith et al. (cited above), and Smith et al., Nature, Vol. 321, pgs. 674-679 (1986). The choice of fluorescent labels is critical in such technology, and represents a major constraint to expanding the capabilities of the technology. Moreover, in spite of the degree of automation achieved, users still must frequently prepare their own labeled oligonucleotide primers for dideoxy-based sequencing. Such primers must be free of sequence failures associated with the synthesis of the oligonucleotide. A standard technique for oligonucleotide purification involves polyacrylamide gel electrophoresis (e.g. Applied Biosystems Users Bulletin, Issue 13, November 9, 1984, "Evaluation and Purification of Synthetic Oligonucleotides"). If a primer has attached to it dye isomers that have close electrophoretic mobilities, primer purification will be difficult, if not impossible. The use of a single isomer simplifies this type of purification.

In the electrophoretic separation of fluorescently labeled oligonucleotides important considerations include the relative synthetic yields and stabilities of the fluorescently labeled oligonucleotide conjugates, and the amount of variability in electrophoretic mobility introduced by the attached label. DNA mobility differences characteristic of the different dyes can be corrected with the proper choice of linker used to join the dye to the oligonucleotide; however, if the individual dyes are present as more than one isomer, then the attached label causes significant spreading of bands, or gives rise to multiple bands of identically sized oligonucleotides which, in turn, leads to spurious sequence determination. Unfortunately, rhodamine dyes, an important class of fluorescent labels used in this technology, are available only as mixtures of isomers, e.g. Haugland, Handbook of Fluorescent Probes and Research Chemicals, (Molecular Probes, Inc. Junction City, Oregon, 1985), and the isomers affect the electrophoretic mobilities of labelled oligonucleotides to different degrees.

In view of the foregoing, the availability of fluorescent labels in pure isomeric forms will increase the applicability of the fluorescent labels, and in particular will increase the sensitivity of fluorescence based methods for distinguishing macromolecules, such as oligonucleotides, separated by gel electrophoresis.

The present invention relates to 5- and 6-sucinimidylcarboxylate isomers of symmetric and asymmetric rhodamine dyes, and to methods of making and using them. Throughout, the Colour Index (Association of

Textile Chemists, 2nd. Ed., 1971) numbering scheme is used to identify the carbon atoms of the rhodamine dyes. Thus, carbon atoms in the xanthene-like structure are identified by primed numbers and carbon atoms of the 9'-substituted phenyl are identified by unprimed numbers as indicated in the general formula I below.

In accordance with the invention, there are now provided isomerically pure 5- and 6-succinimido-N-carboxyl rhodamine dyes and salts thereof.

More particularly, there are provided compounds of the general formula I:

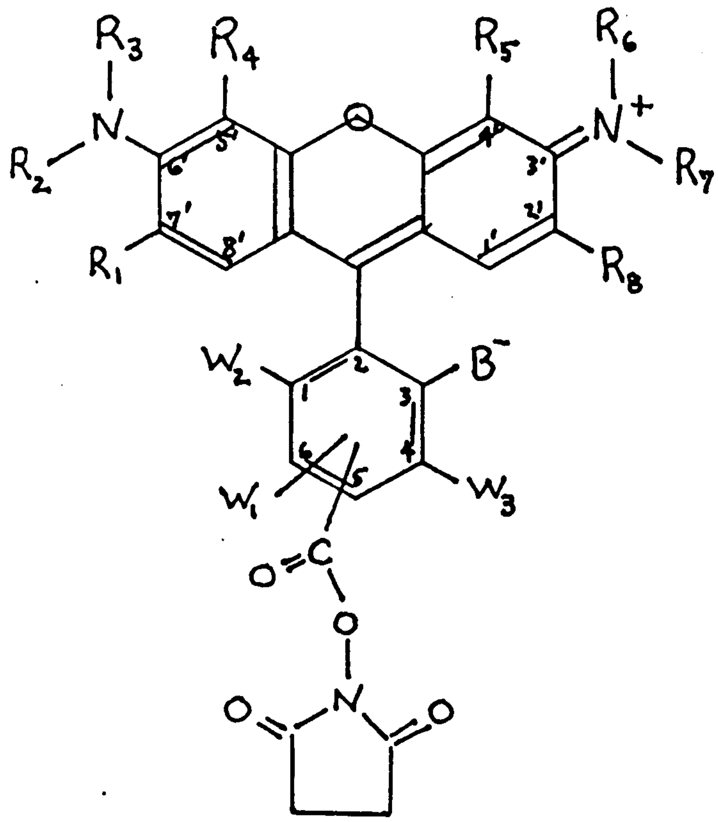

Formula I

wherein:

B is an anionic group, preferably carboxylate or sulfonate, and more preferably carboxylate.

$R_1$ and $R_8$ taken alone are each hydrogen, halogen, alkyl having from 1 to 8 carbon atoms, alkylether having from 1 to 8 carbon atoms, or alkylthioether having from 1 to 8 carbon atoms, and $R_1$ taken together with $R_2$ and $R_8$ taken together with $R_7$ are alkyl chains each having from 2 to 5 carbon atoms connecting the 7′ carbon to the nitrogen attached to the 6′ carbon and connecting the 2′ carbon to the nitrogen attached to the 3′ carbon, respectively. Preferably, $R_1$ and $R_8$ taken alone are each hydrogen, alkyl having from 1 to 3 carbon atoms, chloro, or alkylether having from 1 to 3 carbon atoms, and $R_1$ taken together with $R_2$ and $R_8$ taken together with $R_7$ each form an alkyl chain having from 2 to 3 carbon atoms connecting the 7′ carbon to the nitrogen attached to the 6′ carbon and connecting the 2′ carbon to the nitrogen attached to 3′ carbon, respectively. Most preferably, $R_1$ and $R_8$ taken alone are each hydrogen, and $R_1$ taken together with $R_2$ and $R_8$ taken together with $R_7$ each form an alkyl chain having 3 carbon atoms connecting the 7′ carbon to the nitrogen attached to the 6′ carbon and connecting the 2′ carbon to the nitrogen attached to the 3′ carbon, respectively.

$R_2$ and $R_7$ taken alone are each alkyl having from 1 to 8 carbon atoms, and $R_2$ taken together with $R_1$ and $R_7$ taken together with $R_8$ are each alkyl chains having from 2 to 5 carbon atoms as described above.

Preferably, $R_2$ and $R_7$ taken alone are each alkyl having from 1 to 3 carbon atoms, and $R_2$ taken together with $R_1$ and $R_7$ taken together with $R_8$ are alkyl chains each having from 2 to 3 carbon atoms connecting the 7′ carbon to the nitrogen attached to the 6′ carbon and connecting the 2′ carbon to the nitrogen attached to 3′ carbon, respectively. Most preferably, $R_2$ and $R_7$ taken alone are methyl or ethyl, and $R_2$ taken together with $R_1$ and $R_7$ taken together with $R_8$ are alkyl chains each having 3 carbon atoms connecting the 7′ carbon to the nitrogen attached to the 6′ carbon and connecting the 2′ carbon to the nitrogen attached to the 3′ carbon, respectively.

$R_3$ and $R_6$ taken alone are each alkyl having from 1 to 8 carbon atoms, and $R_3$ taken together with $R_4$ and $R_6$ taken together with $R_5$ are alkyl chains each having from 2 to 5 carbon atoms connecting the 5′ carbon to the nitrogen attached to the 6′ carbon and connecting the 4′ carbon to the nitrogen attached to the 3′ carbon, respectively. Preferably, $R_3$ and $R_6$ taken alone are alkyl each having from 1 to 3 carbon atoms, and $R_3$ taken together with $R_4$ and $R_6$ taken together with $R_5$ form alkyl chains each having from 2 to 3 carbon atoms connecting the 5′ carbon to the nitrogen attached to the 6′ carbon and connecting the 4′ carbon to the nitrogen attached to the 3′ carbon, respectively. Most preferably, $R_3$ and $R_6$ taken alone are methyl or ethyl, and $R_3$ taken together with $R_4$ and $R_6$ taken together with $R_5$ are alkyl chains each having 3 carbon atoms connecting the 5′ carbon to the nitrogen attached to the 6′ carbon and connecting the 4′ carbon to the nitrogen attached to the 3′ carbon, respectively.

$R_4$ and $R_5$ taken alone are hydrogen, alkyl having from 1 to 8 carbon atoms, halogen, alkylether having from 1 to 8 carbon atoms, or alkylthioether having from 1 to 8 carbon atoms, and $R_4$ taken together with $R_3$ and $R_5$ taken together with $R_6$ are alkyl chains each having from 2 to 5 carbon atoms as described above. Preferably, $R_4$ and $R_5$ taken alone are hydrogen, chloro, alkyl having from 1 to 3 carbon atoms, or alkylether having from 1 to 3 carbon atoms, and $R_4$ taken together with $R_3$ and $R_5$ taken together with $R_6$ are alkyl chains each having from 2 to 3 carbon atoms as described above. Most preferably, $R_4$ and $R_5$ taken alone are hydrogen, and $R_4$ taken together with $R_3$ and $R_5$ taken together with $R_6$ are alkyl chains each having 3 carbon atoms connecting the 5′ carbon to the nitrogen attached to the 6′ carbon and connecting the 4′ carbon to the nitrogen attached to the 3′ carbon, respectively.

$W_1$, $W_2$, and $W_3$ are hydrogen or chloro, and preferably hydrogen.

The invention also includes methods for producing 5- and 6-succinimidylcarboxylates of rhodamine dyes. One method comprises the steps of (1) separating the 6-carboxylrhodamine isomer from the 5-carboxylrhodamine isomer, (2) reacting the 6- (or 5-) carboxylrhodamine with equivalent amounts of di-N-succinimidylcarbonate (DSC) and 4-dimethylaminopyridine (DMAP) to form a rhodamine-6- (or -5-) succinimidylcarboxylate in a reaction mixture, and (3) separating the rhodamine-6-(or -5-) succinimidylcarboxylate from the reaction mixture. In another method of the invention a mixture of 5- and 6-succinimidylcarboxylates are formed first, and then separated into isomers. An important feature of the invention is the discovery of a general method for forming N-hydroxysuccinimide (NHS) esters from carboxylic acids of rhodamines by use of stoichiometric amounts of DSC and DMAP.

The invention further includes methods of using the compounds of the invention in gel electrophoretic separations of macromolecules, particularly in gel electrophoretic separations of oligonucleotides on the basis of size. For example, an important technique in molecular biology is the dideoxy chain termination method of DNA sequencing, e.g. Sanger et al. (cited above). There, mixtures of labeled oligonucleotides are formed comprising different size classes of molecules. Members of each size class carry an identical label so that as the molecules are separated from the mixture by gel electrophoresis classes of identically sized oligonucleotides can be detected by their common labels. The use of fluorescent labels consisting of pure carboxylate isomers of rhodamine dyes is required to resolve the different size classes of the oligonucleotides on a gel.

As used herein the terms "rhodamine-X-5-succinimidylcarboxylate" and "rhodamine-X-6-succinimidylcarboxlyate" (abbreviated "5-ROX-NHS" and "6-ROX-NHS", respectively) shall refer to the compounds of Formula I wherein $R_1$ and $R_2$, $R_3$ and $R_4$, $R_5$ and $R_6$, and $R_7$ and $R_8$ are taken together to form 3 carbon alkyl chains as described above, B is carboxylate, and $W_1$, $W_2$, and $W_3$ are hydrogen, and wherein the succinimidylcarboxylate moiety is attached to the 5- and 6- carbons, respectively. 5-ROX and 6-ROX shall refer to 5- and 6- carboxy precursors of these compounds, respectively. As used herein the terms "tetramethylrhodamine-5-succinimidylcarboxylate" and "tetramethylrhodamine-6-succinimidylcarboxylate" (abbreviated "5-TMR-NHS" and "6-TMR-NHS", respectively) shall mean the compounds of Formula I wherein $R_1$, $R_4$, $R_5$, $R_8$, $W_1$, $W_2$, and $W_3$ are hydrogen, B is carboxylate, and $R_2$, $R_3$, $R_6$, and $R_7$ are methyl, and wherein the succinimidylcarboxylate moiety is attached to the 5- and 6- carbons, respectively. 5-TMR and 6-TMR shall refer to the 5- and 6-carboxy precursors of these compounds, respectively.

As used herein in reference to isomeric forms of 5- or 6- carboxylates, or 5- or 6-succinimidylcarboxylates of rhodamine dyes, the term "isomerically pure" means that no detectable double band formation

takes place because of the presence of both isomers when an oligonucleotide labeled by a purified isomer of the rhodamine is separated by gel electrophoresis.

The invention includes N-hydroxysuccinimide esters of 5- and 6-carboxylrhodamines and methods of making and using the same. An important feature of the invention is the reaction condition of having substantially stoichiometric amounts of DSC and DMAP present for esterification of the 5- or 6- forms of the rhodamine dyes to produce high yields at room temperature. The general reaction scheme of the invention is defined by Formula II:

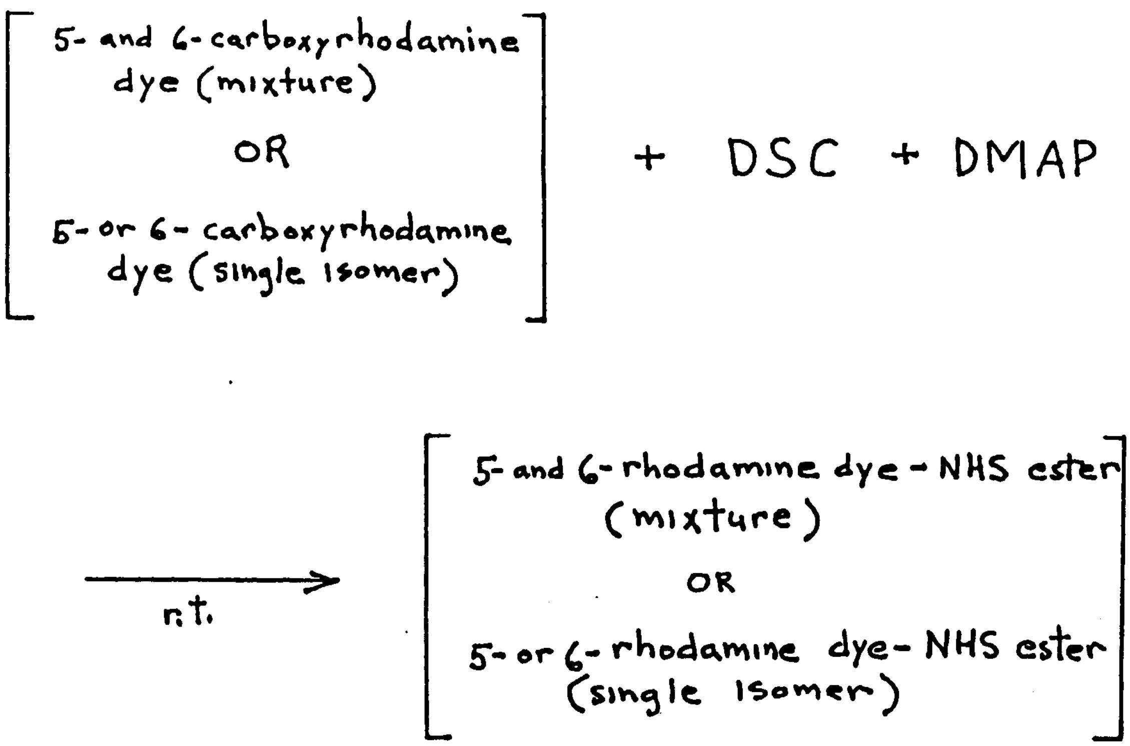


**Formula II**

The methods comprise reacting the acid form of a 5- or 6-carboxylrhodamine (either as a mixture of isomers, or as pure isomers) with equivalent amounts of di-N-succinimidylcarbonate (DSC) and 4-dimethylaminopyridine (DMAP) in a polar aprotic solvent to form the carboxyl N-hydroxysuccinimide (NHS) ester. Suitable polar aprotic solvents include N,N-dimethylformamide (DMF), pyridine, hexamethylphosphoramide (HMPA), or the like. Most preferably, DMF is used as the reaction solvent. The isomerically mixed NHS esters can be separated into their individual isomers for further use. Most preferably, in order to conserve reagents, the acid forms of the 5- or 6-carboxylrhodamines are first separated into their individual isomers by standard separative techniques, e.g. Edmundson et al., Molecular Immunology, Vol. 21, pg.561 (1984), and then the individual 5- or 6- carboxyl isomers are reacted as described above to form the 5- or 6-carboxyl NHS esters, respectively, which are separated from the reaction mixture, again using standard techniques.

Some isomeric mixtures of rhodamine dyes for use with the invention are available commercially, e.g. Eastman Kodak Company (Rochester, New York), Molecular Probes, Inc. (Junction City, OR), and others can be synthesized in accordance with the teachings of U.S. Patents 2,242,572; 2,153,059; 3,822,270; 3,932,415; and 4,005,092.

The following Examples illustrate the invention.

EXAMPLE 1. 6-TMR-NHS

6-TMR acid was separated from a mixture of the 5- and 6-TMR acid isomers by column chromatography. 8.82 mg of 6-TMR acid and 10.5 mg of DSC were dissolved in 0.5 ml of dry DMF under argon. 0.09 ml of a 0.5 molar solution of DMAP in tetrahydrofuran (THF) was added in one portion. After 2 hours at

room temperature, the mixture was taken into 50 ml of chloroform and washed three times with a 1:1 solution of brine:water. The chloroform was evaporated and the residue was purified on a 20 g silica gel column (300:30:8 methylene chloride:methanol:acetic acid elution). Fractions with $R_f$ of about 0.4 were evaporated to dryness, yielding 8.6 mg of 6-TMR-NHS as its acetic acid salt.

EXAMPLE 2. 5-TMR-NHS

5-TMR-NHS was prepared from 82.3 mg of 5-TMR acid, 75 mg of DSC, 0.70 ml of 0.5 molar DMAP in THF in 2 ml dry DMF, as described in Example 1.

EXAMPLE 3. 6-ROX-NHS

6-ROX acid was separated from a mixture of 5- and 6- acid isomers by column chromatography. 46.2 mg of 6-ROX acid and 58 mg of DSC were dissolved in 2 ml of dry DMF under argon and 0.45 ml of a 0.5 molar solution of DMAP in THF was added in one portion. After 1.5 hours at room temperature, the mixture was taken into 100 ml chloroform and washed four times with a 1:1 solution of brine:water. The chloroform was evaporated and the residue was purified on a 40 g silica gel column (300:30:8 methylene chloride:methanol:acetic acid elution). Fractions with $R_f$ of about 0.5 were evaporated to dryness, yielding 56.4 mg of 6-ROX-NHS as its acetic acid salt.

EXAMPLE 4. 5-ROX-NHS

5-ROX-NHS was prepared from 27.4 mg of 5-ROX acid, 30.2 mg of DSC, 0.24 ml of 0.5 molar DMAP in THF in 1.0 ml dry DMF, as described in Example 3.

EXAMPLE 5. 6-TMR Derivatized Aminoethyloligonucleotides

0.50 mg of 6-TMR-NHS was added to a solution consisting of 20 microliters of 1.0 millimolar 5′-aminoethylphosphate oligonucleotide (an 18-mer) in water and 10 microliters of 1 molar $NaHCO_3/Na_2CO_3$ buffer, pH 9.0. After 1 hour in the dark, the solution was passed through a 10 ml Sephadex G-25 (medium) column with 0.1 molar triethylammonium acetate buffer, pH 7.0. The band of colored material eluting in the exclusion volume was collected. Reverse phase HPLC showed greater than 90% conversion of the oligonucleotide to the fluorescent product.

EXAMPLE 6. 5-TMR Derivatized Aminoethyloligonucleotide

5-TMR-NHS was reacted with a 5′-aminoethylphosphate oligonucleotide (18-mer) as described in Example 5. The labeled oligonucleotide was removed from the reaction mixture as described in Example 5. The products from this example and from Example 5 were purified by preparative HPLC, 3′ end-labeled with [alpha-$^{32}$P]-cordycepin-5′-triphosphate via terminal deoxynucleotidyltransferase, and electrophoresed side by side on a 20% polyacrylamide gel. Exposure of the gel onto x-ray film showed that the product from Example 5 moved the equivalent of about 1/2 nucleotide slower on the gel than the product from the present example.

EXAMPLE 7. 6-ROX Derivatized Aminoethyloligonucleotide

To a solution consisting of 20 microliters of 1.0 millimolar 5′-aminoethylphosphate oligonucleotide (18-mer) in water and 10 microliters of 1 molar $NaHCO_3/Na_2CO_3$ buffer, pH 9.0, was added 0.42 mg 6-ROX-NHS, followed by 5 microliters of DMF. After 1 hour in the dark, the solution was passed through a 10 ml Sephadex G-25 (medium) column with 0.1 molar triethylammonium acetate buffer, pH 7.0. The band of colored material eluting in the exclusion volume was collected. Reverse phase HPLC showed greater than 70% conversion of the oligonucleotide to the fluorescent product.

EXAMPLE 8. 5-ROX Derivatized Aminoethyloligonucleotides

5-ROX-NHS was reacted with a 5′-aminoethylphosphate oligonucleotide (18-mer) as described in Example 7. The labeled oligonucleotide was removed from the reaction mixture as described in Example 7. The products from the present example and from Example 7 were purified by preparative HPLC, 3′-end

labeled as in Example 6, and electrophoresed side by side on a 20% polyacrylamide gel. Exposure of the gel onto x-ray film showed that the product from Example 7 moved the equivalent of about 1/2 nucleotide slower than the product of the present example.

EXAMPLE 9. Use of 6-succinimidylcarboxylate Isomers in DNA Sequencing by the Dideoxy Chain Termination Method

DNA sequence analysis is highly useful, both scientifically and commercially. The two primary techniques for sequencing DNA fragments are chemical methods, e.g., Maxam and Gilbert, Proc. Nat. Acad. Sci., Vol. 74, p. 560 (1970), and dideoxy chain termination methods, e.g., Smith, Methods in Enzymology, Vol. 65, Grossman and Moldave, eds., pgs. 560-580 (Academic Press, New York, 1980), and Sanger et al., Proc Natl. Acad. Sci., Vol. 74, pgs. 5363-5367 (1977). The method of the invention can be applied with either technique to substitute fluorescent labels for radioactive labels. In this example, it is shown how the subject invention is used in the dideoxy chain termination method of DNA sequencing. Before the dideoxy chain termination method is described, it will be useful to define the following terms. DNA polymerase is a large multi-function enzyme which catalyzes the synthesis of single-stranded DNA. The particular kind of DNA polymerase used in the method is the so-called Klenow fragment of Escherichia coli DNA polymerase I. This fragment possesses the synthetic function of the enzyme. For synthesis DNA polymerase requires a template, a primer, and a source of deoxyribonucleotides.

A template is a single-stranded piece of DNA which determines the sequence of nucleotides in the single-stranded piece of DNA synthesized by the DNA polymerase. During synthesis, the DNA polymerase moves along the template, and for each nucleotide base thereof, the DNA polymerase attaches the complementary nucleotide to the growing chain of single-stranded DNA. A complementary nucleotide base is one associated with a given base in accordance with the base-pairing rule for the formation of double-stranded DNA. The base-pairing rule requires that adenosine of one strand always be paired with thymidine of the other strand, and that cytidine of one strand always be paired with guanosine of the other strand. Thus, when the DNA polymerase encounters an adenosine on the template, it adds a thymidine to the chain being synthesized, and when it encounters a cytidine, it adds a guanosine. After the DNA polymerase moves on, the newly synthesized chain and the complementary portion of the template are in double-stranded form.

A primer is a fragment of single-stranded DNA. The primer provides a starting location for the DNA polymerase to begin adding nucleotides in the synthesis process. The primer must be annealed to the piece of single-stranded DNA containing the template so that a section of double-stranded DNA is provided as the starting point for the DNA polymerase.

Dideoxyribonucleotides are identical to deoxyribonucleotides except that they lack both the 2′- and 3′-hydroxyl groups on the ribose moiety, instead of just the 2′-hydroxyl as with deoxyribonucleotides. Dideoxyribonucleotides are sometimes referred to as analogs of deoxyribonucleotides, in that DNA polymerase accepts the dideoxy derivatives in place of the corresponding deoxyribonucleotide in the DNA synthesis process. When such a substitution takes place, synthesis stops because the DNA polymerase has no 3′-hydroxyl group on which to attach the subsequent nucleotide.

In the dideoxy chain termination method a DNA strand to be sequenced is used as a template for Escherichia coli DNA polymerase I. A primer is annealed to a piece of single-stranded DNA containing the template, and then it is extended enzymatically up to 400 or more nucleotides in the presence of radioactively labeled deoxyribonucleoside triphosphates, e.g. $^{32}$P-labeled adenosine triphosphate, and the dideoxyribonucleoside triphosphate analog of one of the four nucleotides. That is, four separate reactions are carried out each including a different dideoxy analog. Because DNA chain growth requires the addition of deoxyribonucleotides to the 3'-hydroxyl, incorporation of a dideoxyribonucleotide terminates chain growth. Incorporation of the dideoxy analog in place of the normal nucleotide occurs randomly, so that each of the four reactions generates a heterogeneous population of labeled strands terminating with the same nucleotide, which can be separated electrophoretically according to chain length. That is, four classes of oligonucleotides are established based on the type of terminal dideoxyribonucleoside which is present. A single stranded DNA phage M13 is used to clone copies of the DNA fragment to be sequenced. When a sufficient quantity of M13 is cloned, the M13 DNA is purified and separated into four aliquots. In each aliquot the synthesis or chain growth reaction takes place in the presence of the respective dideoxyribonucleotides.

In accordance with the invention, instead of labeling oligonucleotides by incorporation of radioactive nucleotides during the chain growth phase, primers are synthesized and then labeled by attaching an appropriate linking functionality and reacting it with a dye. An amine linking functionality is attached by

reacting the primers with 2-methoxy-3-trifluoroacetyl-1,3,2-oxazaphosphacyclopentane, described in European Patent Publication No. 0233053A or other phosphoramidite linking agents, e.g. as disclosed by Smith et al. Nucleic Acids Research, (cited above). An important constraint on this variation of the dideoxy chain termination method is that the fluorescent dyes be spectrally resolvable. As used herein the term "spectrally resolvable" means that the fluorescent emission bands of the four dyes are sufficiently non-overlapping so that they can be distinguished using standard photodetection methods. Another important constraint is that the dyes must be electrophoretically compatible. As used herein the term "electrophoretically compatible" means that a selection of linking agents is available for balancing differences in electrophoretic mobilities attributable to the dyes so that a sequence of bands of electrophoretically separated dye-labeled oligonucleotides corresponds to the ordering of the oligonucleotides according to size.

Protecting groups are removed and 6-ROX-NHS reacted with the deprotected 5'-amine as described in Example 7 to form ROX-primer conjugates. In a separate synthesis, protecting groups are removed and 6-TMR-NHS is reacted with the deprotected 5'-amine as described in Example 5 to form TMR-primer conjugates. The primer conjugates are then used in accordance with the dideoxy chain termination method as disclosed by Smith et al. Nucleic Acids Research and Nature (both cited above). Preferably, carboxyfluorescein and 2',7'-dimethoxy-4',5'-dichlorocarboxyfluorescein, attached to the 5'-amines of their respective dideoxy-oligonucleotides via hexyl linkages, are used whenever 6-ROX and 6-TMR are attached via ethyl linkages.

The relative size of the extended primers and the nature of their terminal dideoxyribonucleotides are determined as bands of homogeneously sized extended primers travel down an electrophoresis lane and are detected by a fluorimeter or spectrophotometer after illumination. Preferably, for the above selection of dyes, bands are illuminated with both 514 nm and 488 nm laser light, either sequentially or simultaneously. Homogeneously sized extended primers labeled with either 6-ROX or 6-TMR travel down the electrophoresis gel as narrow bands, while homogeneously sized extended primers labeled with mixtures of 5-ROX and 6-ROX, or 5-TMR and 6-TMR travel down the gel as two bands giving incorrect sequence information.

## Claims

1. Isomerically pure 5- and 6-succinimido-N-carboxyl rhodamine dyes and salts thereof.

2. A compound according to claim 1, having the general formula

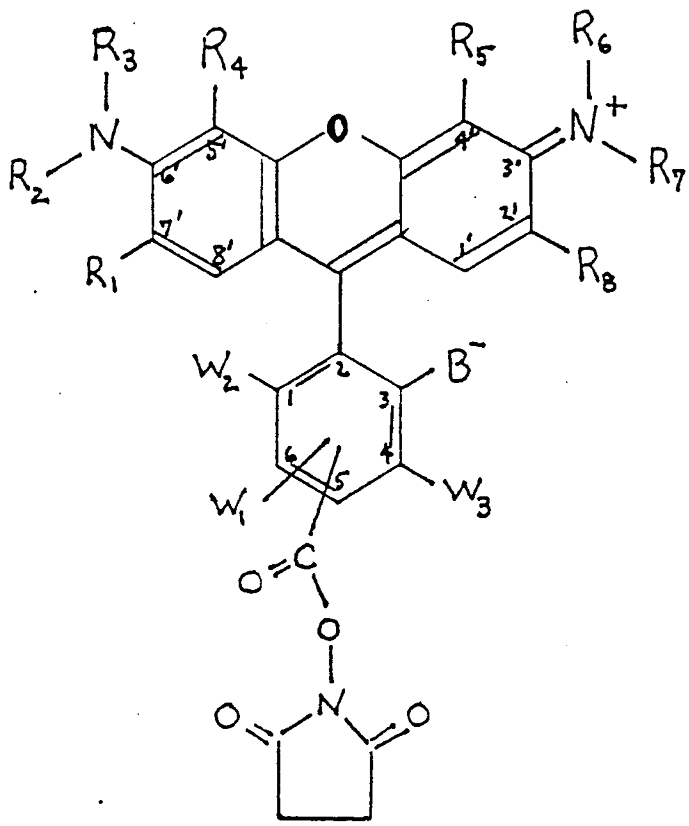

in which

each of $R_1$, $R_4$, $R_5$ and $R_8$ is hydrogen or halogen, or alkyl, alkylether or alkylthioether having from 1 to 8 carbon atoms and each of $R_2$, $R_3$, $R_6$ and $R_7$ is alkyl having from 1 to 8 carbon atoms,

or in which

each of one or more pairs of $R_1$ and $R_2$, $R_3$ and $R_4$, $R_5$ and $R_6$, and $R_7$ and $R_8$ together constitute an alkylene bridging groups having from 2 to 5 carbon atoms,

and each of $W_1$, $W_2$ and $W_3$ is hydrogen or chloro, and B is an anionic group.

**3.** A compound according to claim 2, wherein each pair of $R_1$ and $R_2$, $R_3$ and $R_4$, $R_5$ and $R_6$ and $R_7$ and $R_8$ constitutes an alkylene bridging group each having 3 carbon atoms, each of $W_1$, $W_2$ and $W_3$ is hydrogen, and B is carboxyl.

**4.** A compound according to claim 2, wherein each of $R_1$, $R_4$, $R_5$, $R_8$, $W_1$' $W_2$ and $W_3$ is hydrogen, each of $R_2$, $R_3$, $R_6$ and $R_7$ is methyl, and B is carboxyl.

**5.** A compound according to any one of claims 1 to 4, for use in the fluorescent labelling of nucleotides.

**6.** A method of forming an N-hydroxysuccinimide ester of a rhodamine dye carboxylic acid, comprising reacting a rhodamine dye carboxylic acid selected from isomerically pure 5-carboxylate rhodamine, isomerically pure 6-carboxylate rhodamine, and mixtures of 5- and 6-carboxylate rhodamines with equivalent amounts of di-N-succinimidylcarbonate and 4-dimethylaminopyridine.

**7.** A method according to claim 6, wherein the reaction is carried out in an aprotic polar solvent.

**8.** A method according to claim 6 or claim 7, wherein the reaction is carried out at room temperature.

**9.** A method of determining the nucleotide sequence of a single stranded DNA, the method comprising the steps of:

synthesizing first, second, third, and fourth fluorescently labeled primers having base sequences such that (1) each is labeled with a different fluorescent dye, (2) at least one fluorescent dye is an

isomerically pure 6-carboxylrhodamine or an isomerically pure 5-carboxylrhodamine, and (3) the different fluorescent dyes are spectrally resolvable and electrophoretically compatible;

inserting the single stranded DNA into a single stranded plasmid adjacent to a complementary region to the base sequence of the primers such that the primers are capable of priming the synthesis of a complementary oligonucleotide to the inserted single stranded DNA by a DNA polymerase;

replicating the single stranded plasmid containing the single stranded DNA;

separating the replicated single stranded plasmids into first, second, third and fourth portions;

annealing the first fluorescently labeled primer to the complementary region of the single stranded plasmids of the first portion and reacting the first fluorescently labeled primer and single stranded plasmid with a DNA polymerase in the presence of triphosphates of adenosine, guanosine, cytidine, thymidine, and dideoxyadenosine to form a first set of double stranded DNAs;

annealing the second fluorescently labeled primer to the complementary region of the single stranded plasmids of the second portion and reacting the second fluorescently labeled primer and single stranded plasmid with a DNA polymerase in the presence of triphosphates of adenosine, guanosine, cytidine, thymidine, and dideoxyguanosine to form a second set of double stranded DNAs;

annealing the third fluorescently labeled primer to the complementary region of the single stranded plasmids of the third portion and reacting the third fluorescently labeled primer and single stranded plasmid with a DNA polymerase in the presence of triphosphates of adenosine, guanosine, cytidine, thymidine, and dideoxycytidine to form a third set of double stranded DNAs;

annealing the fourth fluorescently labeled primer to the complementary region of the single stranded plasmids of the fourth portion and reacting the fourth fluorescently labeled primer and single stranded plasmid with a DNA polymerase in the presence of triphosphates of adenosine, guanosine, cytidine, thymidine, and dideoxythymidine to form a fourth set of double stranded DNAs;

forming a mixture of the double stranded oligonucleotides from the first, second, third, and fourth sets of double stranded DNAs;

melting the double stranded DNAs of the mixture of the first, second, third, and fourth sets of double stranded DNAs to form single stranded oligonucleotides; and

separating according to size the single stranded oligonucleotides in the mixture by gel electrophoresis.

**Revendications**

1. Colorants de succinimido-5 et -6 N-carboxyl rhodamine isomériquement purs, et leurs sels.

2. Composé selon la revendication 1, représenté par la formule générale :

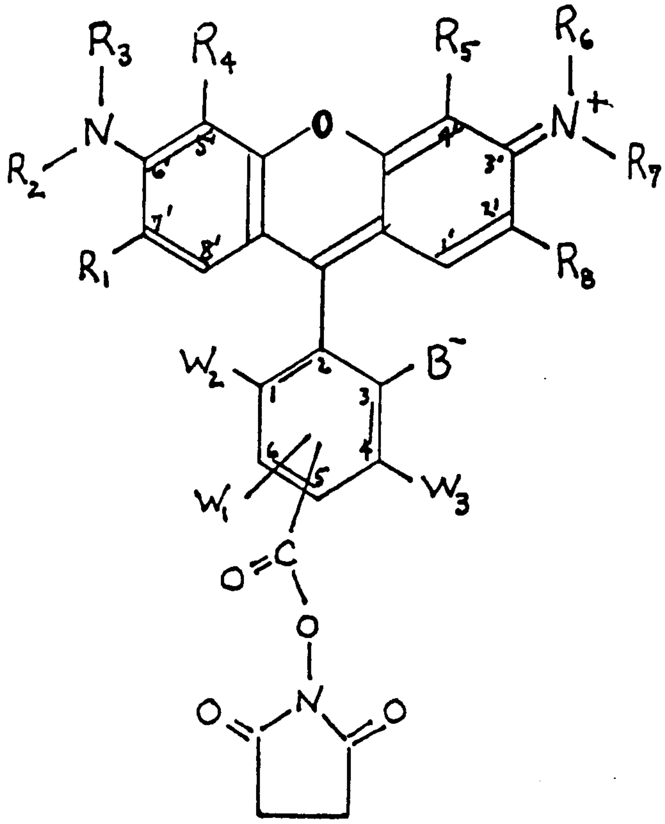

dans laquelle :

- $R_1$, $R_4$, $R_5$ et $R_8$ représentent chacun hydrogène ou halogène, ou alkyle, alkyléther ou alkylthioéther ayant de 1 à 8 atomes de carbone ; et
- R, $R_3$, $R_6$ et $R_7$ représentent chacun alkyle ayant de 1 à 8 atomes de carbone ; ou

dans laquelle :

- une ou plusieurs paires formées par $R_1$ et $R_2$, $R_3$ et $R_4$, $R_5$ et $R_6$, et $R_7$ et $R_8$ définissant chacune un groupe pontant alkylène ayant de 2 à 5 atomes de carbone; et
- $W_1$, $W_2$ et $W_3$ représentent chacun hydrogène ou chloro ; et
- B représente un groupe anionique.

**3.** Composé selon la revendication 2, dans lequel:
- chacune des paires $R_1$ et $R_2$, $R_3$ et $R_4$, $R_5$ et $R_6$, et R, et $R_8$ définit un groupe pontant alkylène ayant chacun 3 atomes de carbone ;
- $W_1$, $W_2$ et $W_3$ représentent chacun hydrogène ; et
- B représente carboxyle.

**4.** Composé selon la revendication 2, dans lequel:
- $R_1$, $R_4$, $R_5$, $R_8$, $W_1$, $W_2$ et $W_3$ représentent chacun hydrogène;
- $R_2$, $R_3$, $R_6$ et $R_7$ représentent chacun méthyle, et
- B représente carboxyle.

**5.** Composé selon l'une quelconque des revendications 1 à 4, pour utilisation dans le marquage fluorescent de nucléotides.

**6.** Procédé de formation d'un ester de N-hydroxysuccinimide d'un colorant rhodamine acide carboxylique comprenant la réaction d'un colorant rhodamine acide carboxylique choisi parmi la carboxylate-5 rhodamine isomériquement pure, la carboxylate-6 rhodamine isomériquement pure, et des mélanges de carboxylate-5 et -6 rhodamines avec des quantités équivalentes de carbonate de di-N-succinimidyle et de diméthylamino-4 pyridine.

7. Procédé selon la revendication 6, dans lequel la réaction est effectuée dans un solvant polaire aprotique.

8. Procédé selon la revendication 6 ou la revendication 7, dans lequel la réaction est effectuée à la température ambiante.

9. Procédé pour la détermination de la séquence nucléotidique d'un ADN simple brin, le procédé comprenant les étapes consistant à :
   - synthétiser des première, deuxième, troisième et quatrième amorces marquées par un élément fluorescent, ayant des séquences de bases telles que (1) chacune soit marquée par un colorant fluorescent différent, (2) au moins un colorant fluorescent est une carboxyl-6 rhodamine isomériquement pure ou une carboxyl-5 rhodamine isomériquement pure, et (3) les différents colorants fluorescents peuvent être séparés par résolution spectrale et électrophorétiquement compatibles ;
   - insérer l'ADN simple brin dans un plasmide simple brin au voisinage d'une région complémentaire de la séquence de bases des amorces de telle sorte que les amorces soient capables d'amorcer la synthèse d'un oligonucléotide complémentaire sur l'ADN simple brin inséré par une ADN polymérase ;
   - répliquer le plasmide simple brin contenant l'ADN simple brin ;
   - séparer les plasmides simple brin répliqués en des première, deuxième, troisième et quatrième parties ;
   - amener la première amorce marquée par un élément fluorescent sous une forme bicaténaire avec la région complémentaire des plasmides simple brin de la première partie et faire réagir la première amorce marquée par un élément fluorescent et le plasmide simple brin avec une ADN polymérase en présence de triphosphates d'adénosine, de guanosine, de cytidine, de thymidine et de didésoxyadénosine, pour former un premier ensemble d'ADNs double brin ;
   - amener la deuxième amorce marquée par un élément fluorescent sous une forme bicaténaire avec la région complémentaire des plasmides simple brin de la deuxième partie et faire réagir la deuxième amorce marquée par un élément fluorescent et le plasmide simple brin avec une ADN polymérase en présence de triphosphates d'adénosine, de guanosine, de cytidine, de thymidine et de didésoxyguanosine, pour former un deuxième ensemble d'ADNs double brin ;
   - amener la troisième amorce marquée par un élément fluorescent sous une forme bicaténaire avec la région complémentaire des plasmides simple brin de la troisième partie et faire réagir la troisième amorce marquée par un élément fluorescent et le plasmide simple brin avec une ADN polymérase en présence de triphosphates d'adénosine, de guanosine, de cytidine, de thymidine et de didésoxycytidine, pour former un troisième ensemble d'ADNs double brin ;
   - amener la quatrième amorce marquée par un élément fluorescent sous une forme bicaténaire avec la région complémentaire des plasmides simple brin de la quatrième partie et faire réagir la quatrième amorce marquée par un élément fluorescent et le plasmide simple brin avec une ADN polymérase en présence de triphosphates d'adénosine, de guanosine, de cytidine, de thymidine et de didésoxythymidine, pour former un quatrième ensemble d'ADNs double brin ;
   - former un mélange des oligonucléotides double brin à partir des premier, deuxième, troisième et quatrième ensembles d'ADNs double brin ;
   - fusionner les ADNs double brin du mélange des premier, deuxième, troisième et quatrième ensembles d'ADNs double brin, pour former des oligonucléotides simple brin ; et
   - séparer en fonction de la dimension les oligonucléotides simple brin dans le mélange par électrophorèse sur gel.

**Patentansprüche**

1. Isomerreine 5- und 6-Succinimido-N-carboxylrhodaminfarbstoffe sowie deren Salze.

2. Verbindung nach Anspruch 1 mit der allgemeinen Formel

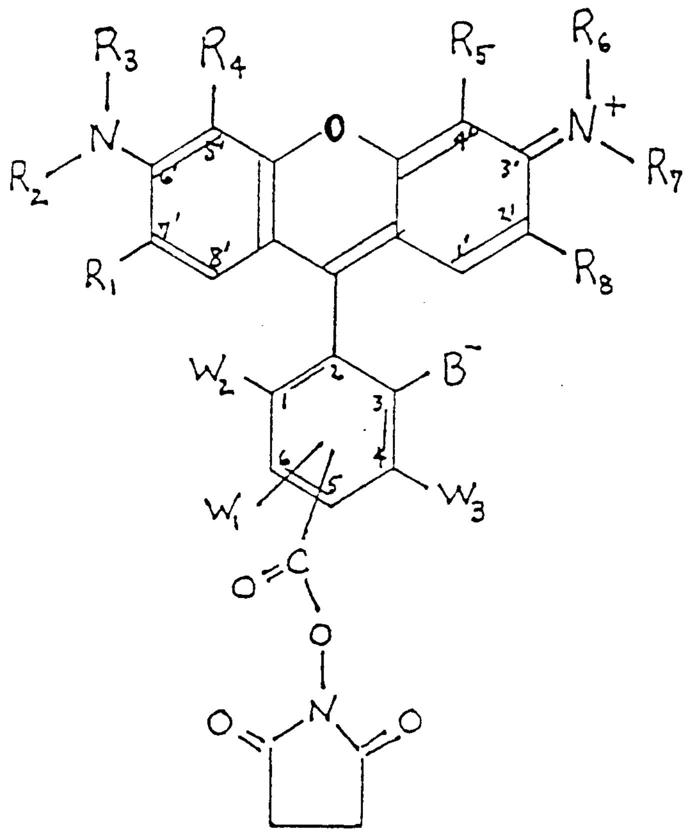

in welcher jeder der Reste $R_1$, $R_4$, $R_5$ und $R_8$ ein Wasserstoff- oder ein Halogenatom oder ein Alkyl-, Alkylether- oder Alkylthioetherrest mit 1 bis 8 Kohlenstoffatomen und jeder der Reste $R_2$, $R_3$, $R_6$ und $R_7$ ein Alkylrest mit 1 bis 8 Kohlenstoffatomen ist,
oder in welcher
jedes von einem oder mehreren Paaren der Reste $R_1$ und $R_2$, $R_3$ und $R_4$, $R_5$ und $R_6$ sowie $R_7$ und $R_8$ gemeinsam eine Alkylenbrücke mit 2 bis 5 Kohlenstoffatomen bildet,
und jeder der Reste $W_1$, $W_2$ und $W_3$ ein Wasserstoff- oder Chloratom und B ein anionischer Rest ist.

3. Verbindung nach Anspruch 2, worin jedes der Paare $R_1$ und $R_2$, $R_3$ und $R_4$, $R_5$ und $R_6$ sowie $R_7$ und $R_8$ eine Alkylenbrücke mit jeweils drei Kohlenstoffatomen bildet, $W_1$, $W_2$ und $W_3$ jeweils ein Wasserstoffatom und B eine Carboxylgruppe ist.

4. Verbindung nach Anspruch 2, worin jeder der Reste $R_1$, $R_4$, $R_5$, $R_8$, $W_1$, $W_2$ und $W_3$ ein Wasserstoffatom, jeder der Reste $R_2$, $R_3$, $R_6$ und $R_7$ eine Methylgruppe und B eine Carboxylgruppe ist.

5. Verbindung nach einem der Ansprüche 1 bis 4, zur Verwendung bei der Fluoreszenzmarkierung von Nukleotiden.

6. Verfahren zur Herstellung eines N-Hydroxysuccinimidesters einer Rhodaminfarbstoffcarbonsäure, wobei eine Rhodaminfarbstoffcarbonsäure, ausgewählt aus isomerreinem 4-Carboxylatrhodamin, isomerreinem 6-Carboxylatrhodamin und Gemischen aus 5- und 6-Carboxylatrhodaminen, mit äquivalenten Mengen von Di-N-Succinimidylcarbonat und 4-Dimethylaminopyridin umgesetzt wird.

7. Verfahren nach Anspruch 6, wobei die Reaktion in einem protonenfreien polaren Lösungsmittel durchgeführt wird.

8. Verfahren nach Anspruch 6 oder 7, wobei die Reaktion bei Raumtemperatur durchgeführt wird.

9. Verfahren zur Bestimmung der Nukleotidsequenz einer Einzelstrang-DNA mit den folgenden Schritten:

Synthetisieren eines ersten, zweiten, dritten und vierten fluoreszenzmarkierten Primers mit Basensequenzen in einer Weise, daß (1) jeder Primer mit einem anderen Fluoreszenzfarbstoff markiert ist, (2) wenigstens ein Fluoreszenzfarbstoff ein isomerreines 6-Carboxylrhodamin oder ein isomerreines 5-Carboxylrhodamin ist, und (3) die verschiedenen Fluoreszenzfarbstoffe nach ihren Spektren auflösbar und elektrophoretisch kompatibel sind;

Einfügen der Einzelstrang-DNA in ein Einzelstrangplasmid angrenzend an einen zu der Basensequenz der Primer komplementären Bereich, so daß die Primer mittels einer DNA-Polymerase die Synthese eines zu der eingefügten Einzelstrang-DNA komplementären Oligonukleotids initiieren können;

Replizieren des die Einzelstrang-DNA enthaltenden Einzelstrangplasmids;

Auftrennen der replizierten Einstrangplasmide in einen ersten, zweiten, dritten und vierten Teil;

Hybridisieren des ersten fluoreszenzmarkierten Primers mit dem komplementären Bereich der Einzelstrangplasmide des ersten Teils und Umsetzen des ersten fluoreszenzmarkierten Primers und des Einzelstrangplasmids mit einer DNA-Polymerase in Gegenwart der Triphosphate von Adenosin, Guanosin, Cytidin, Thymidin und Dideoxyadenosin zur Bildung eines ersten Satzes von Doppelstrang-DNAs;

Hybridisieren des zweiten fluoreszenzmarkierten Primers mit dem komplementären Bereich der Einzelstrangplasmide des zweiten Teils und Umsetzen des zweiten fluoreszenzmarkierten Primers und des Einzelstrangplasmids mit einer DNA-Polymerase in Gegenwart der Triphosphate von Adenosin, Guanosin, Cytidin, Thymidin und Dideoxyguanosin zur Bildung eines zweiten Satzes von Doppelstrang-DNAs;

Hybridisieren des dritten fluoreszenzmarkierten Primers mit dem komplementären Bereich der Einzelstrangplasmide des dritten Teils und Umsetzen des dritten fluoreszenzmarkierten Primers und des Einzelstrangplasmides mit einer DNA-Polymerase in Gegenwart der Triphosphate von Adenosin, Guanosin, Cytidin, Thymidin und Dideoxycytidin zur Bildung eines dritten Satzes von Doppelstrang-DNAs;

Hybridisieren des vierten fluoreszenzmarkierten Primers mit dem komplementären Bereich der Einzelstrangplasmide des vierten Teils und Umsetzen des vierten fluoreszenzmarkierten Primers und des Einzelstrangplasmides mit einer DNA-Polymerase in Gegenwart der Triphosphate von Adenosin, Guanosin, Cytidin, Thymidin und Dideoxythymidin zur Bildung eines vierten Satzes von Doppelstrang-DNAs;

Bilden einer Mischung der Doppelstrangoligonukleotide aus dem ersten, zweiten, dritten und vierten Satz der Doppelstrang-DNAs;

Schmelzen der Doppelstrang-DNAs der Mischung aus dem ersten, zweiten, dritten und vierten Satz der Doppelstrang-DNAs zur Bildung von Einzelstrangoligonukleotiden; und

Trennen der Einzelstrangoligonukleotide in der Mischung nach Größe mittels Gelelektrophorese.